Europäisches Patentamt

European Patent Office

Office européen des brevets

(19)

(11) **EP 0 608 710 B1**

(12) **EUROPÄISCHE PATENTSCHRIFT**

(45) Veröffentlichungstag und Bekanntmachung des Hinweises auf die Patenterteilung:
**30.07.1997 Patentblatt 1997/31**

(51) Int. Cl.$^6$: **C07C 68/06**, C07C 69/96

(21) Anmeldenummer: **94100367.5**

(22) Anmeldetag: **12.01.1994**

(54) **Verfahren zur kontinuierlichen Herstellung von Arylcarbonaten**

Process for the continuous preparation of aryle carbonates

Procédé de préparation continue de carbonates d'aryle

(84) Benannte Vertragsstaaten:
**BE DE ES FR GB IT NL**

(30) Priorität: **25.01.1993 DE 4301899**

(43) Veröffentlichungstag der Anmeldung:
**03.08.1994 Patentblatt 1994/31**

(73) Patentinhaber: **BAYER AG**
**51368 Leverkusen (DE)**

(72) Erfinder:
- **Schön, Norbert, Dr.**
**D-47800 Krefeld (DE)**
- **Rechner, Johann, Dr.**
**D-47800 Krefeld (DE)**
- **Wagner, Paul, Dr.**
**D-40597 Düsseldorf (DE)**
- **Buysch, Hans-Josef, Dr.**
**D-47809 Krefeld (DE)**
- **Gasche, Hans-Erich, Dr.**
**D-51519 Odenthal (DE)**
- **Leiberich, Ricarda, Dr.**
**D-63225 Langen (DE)**

(56) Entgegenhaltungen:
EP-A- 0 461 274           WO-A-92/18458
DE-A- 3 308 921

**Beschreibung**

Die Erfindung betrifft ein kontinuierliches Verfahren zur Herstellung von Arylcarbonaten aus mindestens eine aliphatische Estergruppe enthaltenden Carbonaten und Phenolen einerseits und aus Alkylarylcarbonaten andererseits durch katalysierte Umesterung, wobei die Umsetzung in mindestens zwei hintereinandergeschalteten Rührbehältern durchgeführt wird.

Die Herstellung von aromatischen und aliphatisch-aromatischen Kohlensäureestern (Carbonaten) durch Umesterung, ausgehend von aliphatischen Kohlensäureestern und Phenolen ist im Prinzip bekannt. Dabei handelt es sich um eine Gleichgewichtsreaktion, wobei die Lage des Gleichgewichts fast vollständig in Richtung der aliphatisch substituierten Carbonate verschoben ist. Daher ist es verhältnismäßig leicht, aus aromatischen Carbonaten und Alkoholen aliphatische Carbonate herzustellen. Um die Reaktion im umgekehrten Sinne in Richtung aromatischer Carbonate durchzuführen, ist es notwendig, das sehr ungünstig liegende Gleichgewicht effektiv zu verschieben, wobei nicht nur sehr aktive Katalysatoren, sondern auch eine günstige Verfahrensweise zur Anwendung gelangen müssen.

Zur Umesterung von aliphatischen Kohlensäureestern mit Phenolen sind eine Vielzahl von effektiven Katalysatoren, wie beispielsweie Alkalihydroxide, Lewissäure-Katalysatoren aus der Gruppe der Metallhalogenide (DE-OS 2 528 412 und 2 552 907), Organozinnverbindungen (EP 0 000 879, EP 0 000 880, DE-OS 3 445 552, EP 0 338 760), Bleiverbindungen (JP 57/176 932), Lewis-Säure-/-Protonensäure-Katalysatoren (DE-OS 3 445 553) empfohlen worden.

In den bekannten Verfahren wird die Umesterung in einem chargenweise betriebenen Reaktor drucklos oder unter Druck, gegebenenfalls mit einer zusätzlichen Trennkolonne, durchgeführt. Dabei werden auch mit den aktivsten Katalysatoren Reaktionszeiten von vielen Stunden bis zum Erreichen auch nur mittlerer Umsätze von ungefähr 50 % an Phenol benötigt. So werden bei der chargenweise betriebenen Umesterung von Phenol mit Diethylcarbonat bei 180°C unter Verwendung verschiedener Organozinnverbindungen, wie sie in DE-OS 3 445 552 beschrieben werden, Ausbeuten an Diphenylcarbonat in einer Größenordnung von mehr als 20 % erst nach ca. 24-stündiger Reaktionszeit erreicht; bei der chargenweise betriebenen Umesterung von Phenol und Dimethylcarbonat mit Hilfe von Organozinnkatalysatoren, wie in EP 0 000 879 beschrieben, beträgt der Phenolumsatz nach 30 h 34 % des theoretischen Wertes.

Das bedeutet, daß aufgrund der ungünstigen thermodynamischen Voraussetzungen die beschriebenen, chargenweise betriebenen Umesterungsreaktionen auch bei Verwendung sehr aktiver Katalysatorsysteme im Sinne eines technischen Prozesses nur sehr unvorteilhaft durchführbar sind, da sehr schlechte Raum-Zeit-Ausbeuten und hohe Verweilzeiten bei hohen Reaktionstemperaturen erforderlich sind.

Solche Verfahrensweisen sind auch deshalb besonders unvorteilhaft, da selbst mit sehr selektiven Umesterungskatalysatoren bei den hohen Temperaturen und langen Verweilzeiten von vielen Stunden ein merklicher Anteil an Nebenreaktionen auftritt, beispielsweise die Etherbildung und die Abspaltung von Kohlendioxid.

Es wurde daher versucht, das Reaktionsgleichgewicht durch Adsorption des bei der Umesterung entstehenden Alkohols an Molekularsieben möglichst schnell in Richtung der Produkte zu verschieben (DE-OS 3 308 921). Aus der Beschreibung dieser Verfahrensweise zeigt sich, daß zur Adsorption des Reaktionsalkohols eine große Menge an Molekularsieb benötigt wird, die die Menge an den freiwerdenden Alkoholen noch weit überschreitet. Weiterhin müssen die eingesetzten Molekularsiebe schon nach kurzer Zeit regeneriert werden, und die Umwandlungsrate zu den Alkylarylcarbonat-Zwischenprodukten ist relativ gering. Auch dieses Verfahren erscheint deshalb als technisch und wirtschaftlich nicht vorteilhaft anwendbar.

Aus WO 92/18 458 ist ein kontinuierliches Verfahren zur Herstellung von Diarylcarbonaten in drei Reaktionszonen durch Umesterung von Dialkylcarbonaten mit aromatischen Hydroxy-Verbindungen bekannt, bei dem die Ausgangsverbindungen in die erste Reaktionszone gegeben werden, also in der Gleichstromfahrweise gearbeitet wird.

Ein kontinuierlicher Umesterungsprozeß zur Herstellung von aromatischen Carbonaten, bei dem die Reaktion in einer oder in mehreren mehrstufigen Destillationskolonnen durchgeführt wird, ist in EP-A 0 461 274 beansprucht. Dabei werden zunächst Phenole mit Dialkylcarbonaten zu Arylcarbonatgemischen umgesetzt, die im wesentlichen Alkylarylcarbonate enthalten. In einer zweiten, nachgeschalteten Kolonne werden diese dann zu den gewünschten Diarylcarbonat-Endprodukten weiter umgesetzt. Die Anmelderin betont die Effektivität und die Selektivität ihrer Verfahrensweise. Dagegen stehen die in den Beispielen angegebenen, relativ niedrigen Raumzeit-Ausbeuten der Umsetzung von Phenolen mit Diarylcarbonaten, wobei diese unter optimalen Bedingungen, mit den besten Umesterungskatalysatoren, bei hohen Temperaturen und Drücken erzielt wurden. Die Weiterreaktion der Alkylarylcarbonate zu Diarylcarbonaten verläuft in der angegebenen Verfahrensweise, die aus den Beispielen hervorgeht, im Sinne einer Disproportionierungsreaktion. So ist es nicht verwunderlich, daß bei dieser, im Vergleich zur ersten Umesterungsstufe schnell verlaufenden Reaktion, wesentlich bessere Raumzeitausbeuten erzielt werden.

Ziel einer Verbesserung der Umesterungsreaktion sollte deshalb eine weitere Beschleunigung, vor allem der Umesterungsstufen mit Phenol sein, wobei die Selektivität des gesamten Prozesses nicht herabgesetzt werden sollte.

Überraschenderweise wurde nun gefunden, daß dies in einem kontinuierlich geführten Umesterungsverfahren in Rührkesselkaskaden gelingt, obwohl in EP-A 0 461 274 die besondere Effektivität und die schonenden Bedingungen der Kolonnenfahrweise im Gegensatz zur Kesselfahrweise betont und herausgestellt werden.

Mit der erfindungsgemäßen, kontinuierlichen Verfahrensweise in hintereinandergeschalteten Rührkesseln sind schon bei Normaldruck und wesentlich tieferen Temperaturen, deutlich höhere Raum-Zeit-Ausbeuten der Alkylarylcarbonatbildung erzielbar, als in EP-A 0 461 274 gezeigt. Im Hinblick auf die Argumentation in EP-A 0 461 274 (S.5, Z. 39 ff.) muß aber die Tatsache ganz besonders überraschen, daS diese höheren Umsatzgeschwindigkeiten bei sehr hoher Selektivität der Reaktionen von > 99 % erreicht werden. Die erfindungsgemäße Fahrweise ist auch deshalb als besonders vorteilhaft zu bewerten, weil sehr einfache und leicht beherrschbare Technik mit Standardapparaturen benutzt wird. Die Auslegung solcher Apparate und die Übertragung eines kontinuierlichen betriebenen Rührkesselverfahrens in den technischen Maßstab ist für den Fachmann relativ problemlos durchzuführen- Temperatur, Druck und Verweilzeitspektrum der Reaktionspartner sind leicht über einen weiten Bereich einstellbar, so daß man auch eine variable Verfahrensweise zur Verfügung hat. Der für die endotherm ablaufende Umesterungsreaktion notwendige Wärmeeintrag läßt sich hier problemlos realisieren.

Die Erfindung betrifft demnach ein Verfahren zur Herstellung eines aromatischen Carbonats der Formel

$$R^1\text{-O-CO-O-}R^2 \tag{I}$$

in der

$R^2$      Phenyl oder Naphthyl sowie ein- bis dreifach durch geradkettiges oder verzweigtes $C_1$-$C_4$-Alkyl, geradkettiges oder verzweigtes $C_1$-$C_4$-Alkoxy, Cyano und/oder Halogen substituiertes Phenyl bzw. Naphthyl bedeutet, und

$R^1$      unabhängig von $R^2$ den Bedeutungsumfang von $R^2$ annimmt oder geradkettiges oder verzweigtes $C_1$-$C_6$-Alkyl bedeutet,

durch katalysierte Umsetzung von 0,1 bis 10 mol, bevorzugt 0,2 bis 5 mol, besonders bevorzugt 0,5 bis 3 mol eines organischen Carbonats mit mindestens einer aliphatischen Estergruppe der Formel

$$R^1\text{-O-CO-O-}R^3 \tag{II}$$

in der

$R^3$      geradkettiges oder verzweigtes $C_1$-$C_6$-Alkyl bedeutet und

$R^1$      den obigen Bedeutungsumfang hat,

mit 1 mol einer phenolischen Verbindung der Formel

$$R^2\text{-OX} \tag{III}$$

in der

$R^2$      den obigen Bedeutungsumfang hat und

X      für Wasserstoff oder für -CO-O-$C_1$-$C_6$-Alkyl mit geradkettiger oder verzweigter Alkylgruppe steht,

in Gegenwart eines an sich bekannten Umesterungskatalysators bei 80 bis 350°C, das dadurch gekennzeichnet ist, daß die Umsetzung in mindestens zwei hintereinandergeschalteten Rührbehältern nach dem Prinzip des Querstroms oder des Gegenstroms so durchgeführt wird, daß die phenolische Verbindung der Formel (III) in flüssiger Form in den ersten Rührbehälter und das organische Carbonat der Formel (II) bei Querstromfahrweise in jeden der Rührbehälter und bei Gegenstromfahrweise in den letzten Rührbehälter, eindosiert wird und am letzten Rührbehälter, gegebenenfalls nach Durchlaufen einer Verweilzeitstrecke, das Reaktionsprodukt der Formel (I) in flüssiger Form und die Produkte der Formel

$$R^3\text{-OX} \tag{IV}$$

in der

$R^3$ und X      die genannte Bedeutung haben,

bei Querstromfahrweise am Kopf jedes Rührbehälters und bei Gegenstromfahrweise am Kopf des ersten Rührbehälters entnommen werden.

Die Umesterung nach dem erfindungsgemäßen Verfahren umfaßt mehrere Reaktionen, wie die folgenden Gleichungen in verallgemeinerter Form zeigen (Alk=Alkyl; Ar=Aryl):

$$Alk\text{-}O\text{-}CO\text{-}O\text{-}Alk + Ar\text{-}OH \rightarrow Alk\text{-}O\text{-}CO\text{-}O\text{-}Ar + Alk\text{-}OH \qquad \text{(Gleichung 1)}$$

$$Alk\text{-}O\text{-}CO\text{-}O\text{-}Ar + Ar\text{-}OH \rightarrow Ar\text{-}O\text{-}CO\text{-}O\text{-}Ar + Alk\text{-}OH \qquad \text{(Gleichung 2)}$$

$$2\ Ar\text{-}OCOO\text{-}Alk \rightarrow Ar\text{-}OCOO\text{-}Ar + Alk\text{-}OCOO\text{-}Alk \qquad \text{(Gleichung 3)}$$

Bei der Bildung eines Diarylcarbonats erfolgt die Umesterung von den aliphatischen zu den aromatischen Estergruppen in zwei Stufen, wobei ein Alkylarylcarbonat im Sinne der Gleichung 1 als Produkt der ersten Umesterungsstufe durchlaufen wird.

Die Gleichung 3 zeigt ferner eine Disproportionierungsreaktion, in welcher aus einem gemischten Alkylarylcarbonat sowohl das symmetrische Dialkylcarbonat als auch das gewünschte symmetrische Diarylcarbonat entstehen. Es ist ferner möglich, das Alkylarylcarbonat als das gewünschte Reaktionsprodukt zu erhalten, also nur die erste Umesterungsstufe zu betreiben. Noch weiterhin ist möglich, durch Einsatz von Gemischen verschiedener Phenole auch unsymmetrische Diarylcarbonate zu erhalten.

Zum Einsatz gelangen Dialkylcarbonate mit gleichen oder verschiedenen aliphatischen Estergruppen mit geradkettigem oder verzweigtem $C_1\text{-}C_6$-Alkyl. Solche Dialkylcarbonate sind dem Fachmann bekannt und können nach bekannten Verfahren hergestellt werden. Aus Gründen der Verbilligung wird man im allgemeinen von symmetrischen Dialkylcarbonaten ausgehen.

Geradkettiges oder verzweigtes $C_1\text{-}C_6$-Alkyl ist beispielsweise Methyl, Ethyl, Propyl, Isopropyl, Butyl, Isobutyl, Pentyl oder Hexyl, bevorzugt Methyl oder Ethyl, besonders bevorzugt Methyl.

Geradkettiges oder verzweigtes $C_1\text{-}C_4$-Alkoxy ist beispielsweise Methoxy, Ethoxy, Propoxy, Isopropoxy, Butoxy oder Isobutoxy, bevorzugt Methoxy.

Halogen ist beispielsweise Fluor, Chlor oder Brom, bevorzugt Fluor oder Chlor, besonders bevorzugt Chlor.

Die aromatische Estergruppe kann von einem Phenol oder einem Naphthol, bevorzugt von einem Phenol abgeleitet sein und in der angegebenen Weise ein- bis dreifach, bevorzugt ein- oder zweifach, besonders bevorzugt einfach substituiert sein. Der Cyano-Substituent tritt in der Regel nur einfach als Substituent auf, Ganz besondere Bedeutung hat das erfindungsgemäße Verfahren für die Umesterung von nicht substituiertem Phenol,

Erfindungsgemäß einsetzbare Phenole, die unter die Formel (III) fallen, wenn X für Wasserstoff steht, sind beispielsweise nicht substituiertes Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol und 2-Naphthol.

Bevorzugt einsetzbare phenolische Verbindungen sind demnach allgemein solche der Formel

$$R^{12}\text{-}OH \qquad \text{(V),}$$

in der

$R^{12}$    Phenyl oder einfach durch $C_1\text{-}C_4$-Alkyl, $C_1\text{-}C_4$-Alkoxy oder Chlor substituiertes Phenyl bedeutet.

Hierunter ist das nicht substituierte Phenol besonders bevorzugt.

Als organische Carbonate mit mindestens einer aliphatischen Estergruppe werden bevorzugt symmetrische Dialkylcarbonate der Formel

$$R^3\text{-}O\text{-}CO\text{-}O\text{-}R^3 \qquad \text{(VI),}$$

in der

$R^3$    die angegebene Bedeutung hat,

eingesetzt.

Erfindungsgemäß einsetzbare Dialkylcarbonate sind beispielsweise Dimethylcarbonat, Diethylcarbonat, Dipropylcarbonat, Dibutylcarbonat und Dihexylcarbonat. Bevorzugt einsetzbare Dialkylcarbonste sind Dimethyl-und Diethylcarbonat, besonders bevorzugt Dimethylcarbonat.

Das organische Carbonat (II) mit mindestens einer aliphatischen Estergruppe kann als solches im erfindungsgemäßen Verfahren eingesetzt werdend Es ist jedoch möglich und stellt eine bevorzugte Variante dar, dieses organische Carbonat im Gemisch mit geringen Mengen des zugrundeliegenden Alkohols $R^3\text{-}OH$ einzusetzen. Der Alkohol $R^3\text{-}OH$

tritt im erfindungsgemäßen Verfahren als Spaltprodukt auf und stellt den Spezialfall der Formel (IV) mit X = H dar. Die Spaltprodukte Carbonat (X = -CO-O-$C_2$-$C_6$-Alkyl) und Alkohol (X = H) brauchen demnach zu einer Rückführung des Carbonats in das erfindungsgemäße Verfahren nicht vollständig getrennt zu werden; dies stellt einen energetischen Vorteil dar, Die Menge des im Gemisch mit dem Carbonat zulässigen Alkohols beträgt 0-5 Gew.-%, bevorzugt 0,1-3 Gew.-%, besonders bevorzugt 0,15-2 Gew.-%, bezogen auf die Menge des eingesetzten Carbonats` Die Untergrenze Null bezeichnet die Fahrweise mit reinem Carbonat.

Erfindungsgemäß herstellbare Diarylcarbonate sind beispielsweise Diphenylcarbonat, die symmetrisch und unsymmetrisch substituierten isomeren Bis(methylphenyl)-carbonate, die symmetrisch und unsymmetrisch substituierten isomeren Bis(chlorphenyl)-carbonate, die symmetrisch und unsymmetrisch substituierten isomeren Bis(methoxyphenyl)-carbonate, die symmetrisch und unsymmetrisch substituierten isomeren Bis(ethoxyphenyl)-carbonate, Bis(2,6-dimethylphenyl)-carbonat, Bis(2,4-dimethylphenyl)-carbonat, Di-1-naphthyl-carbonat und Di-2-naphthyl-carbonat, außerdem weitere unsymmetrisch substituierte Diarylcarbonate, beispielsweise die isomeren (Methylphenyl)-phenyl-carbonate, die isomeren (Chlorphenyl)phenyl-carbonate, die isomeren (Methoxyphenyl)phenyl-carbonate, die isomeren Naphthyl-phenyl-carbonate und 1-Naphthyl-2-naphthyl-carbonat.

Bevorzugte erfindungsgemäß herstellbare Diarylcarbonate sind solche der Formeln

$$R^{15}\text{-OCOO-}R^{12} \tag{VII},$$

bzw.

$$R^{12}\text{-OCOO-}R^{12} \tag{VIII},$$

in denen

$R^{12}$ und $R^{15}$ unabhängig voneinander den für $R^{12}$ weiter oben angegebenen Bedeutungsumfang haben.

Besonders bevorzugt herstellbares Diarylcarbonat ist Diphenylcarbonat.

Erfindungsgemäß herstellbare Alkylarylcarbonate sind beispielsweise $C_1$-$C_6$-Alkyl-phenyl-carbonate, wie Methyl-phenyl-carbonat, Ethyl-phenyl-carbonat, Propyl-phenyl-carbonat, Butyl-phenyl-carbonat und Hexyl-phenyl-carbonat, $C_1$-$C_6$-Alkyl(o-, m-, p-methylphenyl)-carbonate, wie Methyl-(o-methylphenyl)-carbonat, Methyl-(p-methylphenyl)-carbonat, Ethyl-(o-methylphenyl)-carbonat, Ethyl-(p-methylphenyl)-carbonat, $C_1$-$C_6$-Alkyl-(o-, m-, p-Chlorphenyl)-carbonate, wie Methyl- oder Ethyl-(p-chlorphenyl)-carbonat und analoge Verbindungen. Besonders bevorzugt herstellbare Alkylarylcarbonate sind Methyl-phenyl-carbonat und Ethyl-phenyl-carbonat, ganz besonders bevorzugt Methyl-phenyl-carbonat.

Zur Vermischung der Reaktionskomponenten sind die erfindungsgemäß zu verwendenden Rührbehälter mit dafür brauchbaren Rührwerkzeugen ausgestattet. Solche Rührer sind dem Fachmann bekannt. Es seien beispielhaft genannt: Scheiben-, Impeller-, Propeller-, Schaufel-, MIG- und Intermig-Rührer, Rohrrührer und andere Hohlrührertypen. Bevorzugte Rührer sind solche, die eine effektive Vermischung von Gasen und Flüssigkeiten erlauben, beispielsweise Hohlrührer, wie Rohrrührer und Dreikantrührer, Propellerrührer, Turbinenrührer etc.

Zur besseren Vermischung können die Rührbehälter bevorzugt mit Strömungsbrecher-Einbauten versehen sein. Diese Strömungsbrecher können gleichzeitig thermostatisierbar zum Einbringen oder zum Abführen von Wärme aus dem Reaktor ausgelegt sein.

Die für die Reaktion notwendige Reaktionswärme kann mit den Edukten eingebracht werden. Es ist jedoch bevorzugt, zusätzliche Energie in den Reaktor beispielsweise über eine Mantelbeheizung oder durch innenliegende Beheizungselemente einzubringen.

Die jeweiligen Rührbehälter können mit Temperaturmeßstellen, Probenahmestellen und anderen Meß- und Regelelementen ausgestattet sein.

Erfindungsgemäß werden mindestens zwei, beispielsweise 2 bis 10 hintereinandergeschaltete Rührbehälter verwendet. Es ist jedoch bevorzugt, 3 bis 10 und besonders bevorzugt, 3 bis 8 hintereinandergeschaltete Rührbehälter zu benutzen.

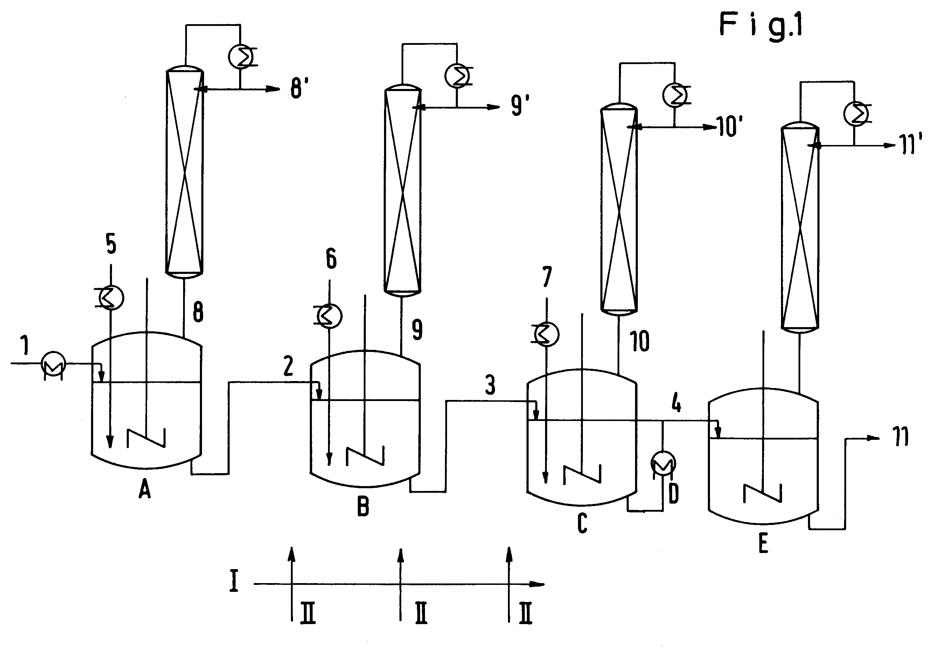

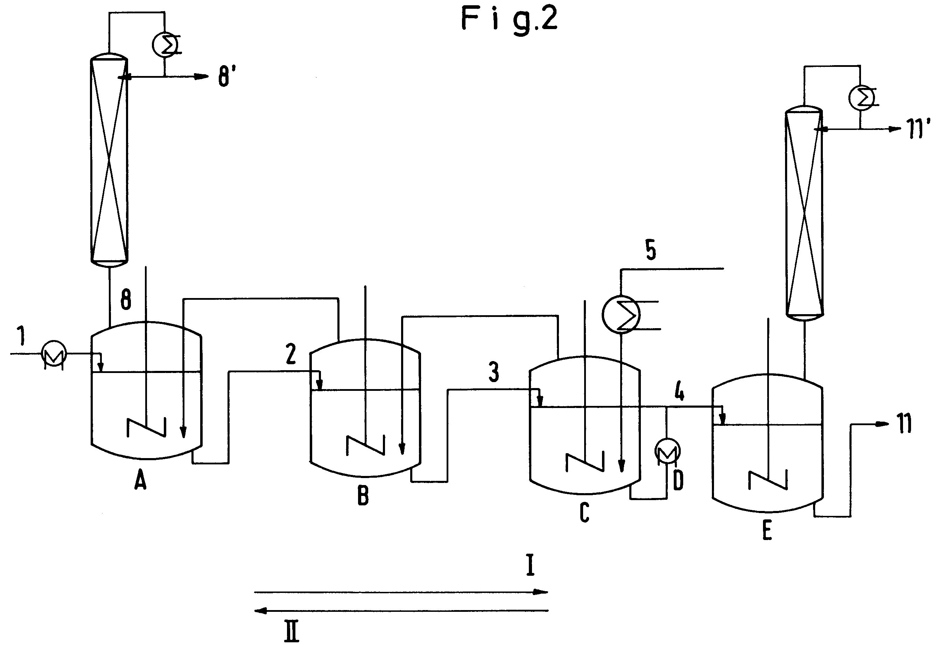

In Fig. 1 und 2 sind beispielhaft verschiedene Ausführungsformen der Erfindung gezeigt. Im Text angegebene Nummern und Buchstaben beziehen sich auf diese Abbildungen.

Die am ersten Rührbehälter A einzudosierende Reaktionskomponente der Formel (III) kann gegebenenfalls in einem vorgeschalteten Erhitzerelement auf die vorgesehene Reaktionstemperatur vorgeheizt werden. So kann in flüssiger Form an beliebiger Stelle, z.B. über Leitung (1) in Fig. 1 oder 2 oder durch ein Rohr (5) in Fig. 1 in die Flüssigphase unterhalb des Rührers eingebracht werden.

Die aus dem jeweiligen Rührbehälter zu entnehmende Flüssigphase kann an geeigneter Stelle des Behälters z.B. im oberen Drittel oder über einen gegebenenfalls beheizbaren, in der Höhe verstellbaren Siphon (2) und (3) in Fig. 1 und 2 entnommen und in den jeweils folgenden Rührbehälter B bzw. C geleitet werden. Dabei soll ein vorgegebener Füllstand im jeweiligen Behälter realisiert werden. Die für den kontinuierlichen Betrieb von Kesselkaskaden zu verwendenden Techniken sind Stand der Technik und dem Fachmann bekannt.

Das organische Carbonat der Formel (II) wird gasförmig durch den kontinuierlich laufenden Flüssigkeitsstrom I entweder im Querstrom (Kreuzstrom) (Fig. 1) oder bevorzugt im Gegenstrom (Fig. 2) geschickt.

Querstrom (Kreuzstrom) bedeutet dabei, daß die Edukte der Formel (II) jeweils an jedem Rührbehälter A, B, und C eindosiert werden ((5), (6), (7) in Fig. 1) und jeweils am Kopf eines jeden Rührbehälters ((8), (9), (10) in Fig. 1) wieder entnommen werden, d.h. die Edukte der Formel (II) durchströmen die Rührbehälter quer zur Flußrichtung der Flüssigphase. Dabei kann die Gesamtmenge der einzudosierenden Edukte der Formel (II) beliebig auf die einzelnen Rührbehälter aufgeteilt werden,

Die bevorzugt zu verwendende Gegenstromfahrweise (Fig. 2) bedeutet, daß die Edukte der Formel (II) am letzten Rührbehälter (C in Fig. 2) über Leitung (5) eindosiert, kontinuierlich gegen die vom ersten zum letzten Rührbehälter laufende Flüssigphase geführt und am Kopf des ersten Rührbehälters (A in Fig. 2) über Leitung (8) entnommen werden.

Die Edukte der Formel (II) können in beiden Fällen entweder flüssig eindosiert und durch die vorhandene Flüssigphase verdampft werden oder bevorzugt in einem vorgeschalteten Apparat verdampft und gasförmig in den jeweiligen Rührbehälter eingebracht werden, Die Eindosierung kann dabei entweder in die Flüssigphase oder in den überstehenden Gasraum erfolgen. Die Durchmischung der Gas- und Flüssigphase wird durch geeignete, oben genannte, dem Fachmann bekannte Rührorgane erreichte Bei der Verwendung von Begasungsrührern mit Hohlwelle kann die Eindosierung der Gasphase auch direkt in die Hohlwelle des Begasungsrührers erfolgen.

Die am Kopf des jeweiligen Rührbehälters zu entnehmenden Reaktionsprodukte der Formel (IV) können z.B. gasförmig über (8'), (9'), (10') und (11') abgenommen werden.

Dabei ist es gegebenenfalls von Vorteil, durch geeignete Dephlegmierung oder durch eine aufgesetzte Kolonne höhersiedende Reaktionsbestandteile, z.B. Produkte der Formel (I) oder Edukte der Formel (III), vorher abzutrennen und in den jeweiligen Rührbehälter zurückzuführen. Die Produkte der Formel (IV) können so beispielsweise ohne Kondensation in eine geeignete Trennvorrichtung eingebracht werden. Dies kann im Falle der Umsetzung von Dimethylcarbonat mit Phenol eine Druckdestillationskolonne zur Trennung des anfallenden Dimethylcarbonat-Methanol-Gemisches sein, wie sie dem Fachmann bekannt ist. Das dabei anfallende Dimethylcarbonat, das gegebenenfalls noch geringe Mengen Methanol enthält, kann als Edukt der Formel (II) in den Umesterungsprozeß zurückgeführt werden.

Ebenso ist es möglich, die Produkte der Formel (IV), gegebenenfalls nach Abtrennung von höhersiedenden Reaktionsbestandteilen, wie oben beschrieben, abzunehmen und zu kondensieren. Eine Reinigung und Auftrennung des Produktstroms kann dann in geeigneter, dem Fachmann bekannter Art und Weise, durchgeführt werden.

Der am letzten Reaktor C flüssig zu entnehmende Produktstrom kann gegebenenfalls in einem nachgeschalteten Abtriebsteil D von leichtsiedenden Bestandteilen, z.B. den Edukten der Formel (II) oder den Produkten der Formel (IV), abgetrennt werden, wobei diese in den letzten Rührbehälter C zurückgeführt werden. Der flüssig entnommene Produktstrom kann nach üblichen Methoden, z.B. durch Destillation aufgearbeitet und gereinigt werden.

In einer weiteren Verfahrensweise kann der flüssig zu entnehmende Produktstrom in 1 bis 5, bevorzugt 1 bis 3 nachgeschaltete gegebenenfalls gerührte oder mit Inertgas begaste Verweilzeitbehälter E geleitet werden, wobei dort weitere Reaktionen im Sinne von Gleichung 2 und/oder Gleichung 3 ablaufen können. In diesem Falle werden das aromatische Carbonat der Formel (I) bei (11) und in E entstandene flüchtige Reaktionsprodukte bei (11') entnommen.

Der jeweils letzte Verweilzeitbehälter E kann gegebenenfalls ein nachgeschaltetes Abtriebsteil besitzen, mit dem leichtsiedende Produkte der Formel (IV) und/oder nicht umgesetzte Edukte der Formel (III) ganz oder teilweise in diesen Verweilzeitbehälter E zurückgeführt werden. Ebenso ist es gegebenenfalls von Vorteil, die am Kopf des ersten Verweilzeitbehälters E z.B. über (11') zu entnehmenden flüchtigen Reaktionsprodukte der Formel (IV) durch ein dort aufgesetztes Verstärker- oder Dephelegmatorteil von höhersiedenden Produkten der Formel (I) oder Edukten der Formel (III) abzutrennen und diese in E zurückzuführen.

In einer weiteren Variante ist der Verweilzeitbehälter E in Form einer Destillationsapparatur ausgebildet, die im Sinne einer "Reaktionsdestillation" betrieben wird, das heißt, daß simultan zur ablaufenden Reaktion eine Destillation der beteiligten Stoffe durchgeführt wird.

Die für eine "Reaktionsdestillation" im Sinne der Erfindung wesentlichen Merkmale sind die folgenden: Das noch nicht umgesetzte Alkylarylcarbonat-Zwischenprodukt wird durch einen speziell gewählten Temperaturgradienten in der Destillationsapparatur weitgehend daran gehindert, den Reaktionsteil des Reaktors nach oben oder nach unten zu verlassen. Die leichtflüchtigen Reaktionsprodukte der Formel (IV) werden am Kopf der Kolonne, das schwerflüchtige Reaktionsprodukt, hier das Diarylcarbonat, wird am Fuß der Kolonne entnommen. Gegebenenfalls vorhandenes, überschüssiges Phenol kann entweder zusammen mit den Diarylcarbonat-Endprodukten am Fuß der Destillationsapparatur oder zusammen mit den Leichtsiederprodukten am Kopf der Apparatur entnommen werden.

Der als "Reaktionskolonne" bezeichnete Reaktor besteht aus einem kolonnenartigen Rohr dem ein Temperaturprofil angelegt wird, das von oben nach unten gesehen ansteigend einen Temperaturbereich von 60 bis 320°C, bevorzugt 65 bis 305°C und besonders bevorzugt von 65 bis 250°C, umfaßt. Zur Einstellung der Temperaturgradienten in den einzelnen Abschnitten des kolonnenartigen Reaktors können diese Abschnitte mit einer Isolierung bzw. einer Thermostatisierung versehen werden. Die Thermostatisierung kann hierbei je nach Bedarf eine Heizung oder eine Kühlung darstellen. Die Reaktionskolonne kann in verschiedenen Abschnitten ihrer Gesamtlänge, entsprechend den Gas- und Flüssigbelastungen und den benötigten Verweilzeiten aufgeweitet oder verengt sein.

Für den mittleren Teil der Reaktionskolonne, den Reaktionsbereich, sind feste Einbauten bevorzugt, für die Teile, in denen Trennungen stattfinden, dagegen Füllkörper und feste Packungen.

Am unteren Ende der Reaktionskolonne sind ein oder mehrere, gegebenenfalls durch adiabatisch isolierte Kolonnenteile getrennte Verdampfer angeordnet. Diese Verdampfer können innerhalb oder bevorzugt außerhalb der Kolonne angeordnet sein. In einer technischen Ausführung werden in der Technik übliche Apparate wie Umlaufverdampfer, Fallfilmverdampfer und Wendelrohrverdampfer verwendet.

Oberhalb der Verdampferzone, in dem als "Reaktionszone" bezeichneten mittleren Bereich, werden bevorzugt feste Einbauten und besonders bevorzugt solche mit großem Flüssigkeits-Holp Up benutzt, beispielsweise Glockenböden mit hohen Überlaufwehren, wie in DE 2 503 195 beschrieben. Die theroretische Bodenzahl in diesem Bereich beträgt 1 bis 50, bevorzugt 2 bis 25 und besonders 2 bis 15.

Wiederum oberhalb dieses Bereichs ist die Kolonne mit weiteren, im besonderen Maße für destillative Stofftrennungen geeigneten Füllkörpern oder Einbauten ausgestattet. Am oberen Ende der Kolonne ist bevorzugt ein Verstärkerteil angeordnet, mit dem ein gezielter Rücklauf der Kolonne einstellbar ist.

Die Reaktionskolonne wird so betrieben, daß man oberhalb der "Reaktionszone" den aus der Rührbehälterkaskade flüssig entnommenen Produktstrom flüssig eindosiert. Dieser Strom durchläuft die "Reaktionszone" und wird dort teilweise in Diarylcarbonat verwandelt, und die noch nicht umgesetzten Reaktanden werden mit Hilfe der beschriebenen Verdampfer gasförmig zurück in die Reaktionszone und die oberen Teile der Kolonne transportiert. Diese kondensieren dort und setzen sich erneut zum Diarylcarbonat-Endprodukt um. Das Diarylcarbonat-Endprodukt wird als höchst siedende Reaktionskomponente im Sumpfbereich der Kolonne angereichert und dort zusammen mit gegebenenfalls homogen gelöstem Katalysator und geringen Mengen Alkylphenylcarbonat und aromatischer Hydroxyverbindung ausgespeist.

Die leichtflüchtigen Reaktionsprodukte der Formel (IV) werden am Kopf der Kolonne entnommen. Die im Überschuß vorhandenen oder nicht umgesetzten Phenole der Formel (III) können entweder am Fuß der Kolonne zusammen mit dem Diarylcarbonat-Endprodukt der Formel (I) oder in einer bevorzugten Fahrweise zusammen mit den Leichtsiederprodukten am Kopf der Kolonne ausgespeist werden.

Der am letzten Reaktor C oder Verweilzeitbehälter E flüssig entnommene Produktstrom, der die Produkte der Formel (I) enthält, kann in einer weiteren besonderen Ausführung der Erfindung, nach Zwischenlagerung in geeigneten Behältern, anstelle des Edukts der Formel (III) zurück in den 1. Reaktorbehälter A gespeist werden. Dies ist gegebenenfalls auch mehrfach möglich, wobei die Zuspeisung des zweiten Edukts der Formel (II) gegebenenfalls auch unterbleiben kann. Zur kontinuierlichen Durchführung einer solchen Fahrweise sind entweder mindestens zwei Lagerbehälter oder ein Lagerbehälter mit mindestens zwei Kammern notwendig, wobei in der 1. Kammer das Produkt aus der laufenden Umsetzung eingespeist und aus der 2. Kammer das Edukt für die laufende Umsetzung entnommen wird Wenn eine Kammer geleert bzw. eine Kammer gefüllt ist, wird die 2. Kammer zur Aufnahme des Produktes aus der Kesselkaskade und die 1. Kammer zur Einspeisung des Eduktes in die Kesselkaskade benutzt.

In einer weiteren Verfahrensweise kann zusätzlich zu den Edukten ein unter den Reaktionsbedingungen inertes Lösungsmittel oder Gas an beliebiger Stelle der Apparatur eingespeist werden. Solche inerte Lösungsmittel sind beispielsweise Kohlenwasserstoffe, wie Hexan, Heptan, i-Octan, Cyclohexan, Methylcyclohexan, Toluol, Xylole, Chlorbenzole, Tetralin, Dekalin etc. Als inerte Gase kommen beispielsweise Kohlendioxid, Stickstoff, Edelgase etc. in Frage.

Die zu verwendenden und als solche bekannten Umesterungskatalysatoren werden bevorzugt zusammen mit den flüssig zu dosierenden Edukten der Formel (III) in gelöster oder suspendierter Form in die Rührbehälter eingebracht. Alternativ kann der Katalysator auch separat oder gelöst bzw. suspendiert in einer geringen Menge des Eduktes der Formel (III) oder in einem systemfremden, geeigneten, inerten Lösungsmittel s.o. eindosiert werden. Im Falle der Verwendung heterogener Katalysatoren können diese auch im Rührbehälter direkt ortsfest eingesetzt werden.

Durch geeignete Filtriervorrichtung muß dabei das Austragen der Katalysatoren verhindert werden.

Wichtig ist, daß mindestens in 2 Rührbehältern ein Katalysator vorhanden ist.

Im Falle der Verwendung von nicht ortsfesten Katalysatoren ist es möglich, die Katalysatoren nach teilweiser oder vollständiger Abtrennung von den Produkten oder Edukten wieder wie oben beschrieben in den Reaktionsprozeß zurückzuführen, wobei gegebenenfalls ein der desaktivierten Katalysatormenge entsprechender Anteil des Katalysators abgetrennt und durch frischen Katalysator ersetzt wird.

Das erfindungsgemäße Verfahren wird bei Temperaturen in der Flüssigphase von 80 bis 350°C, bevorzugt bei 100 bis 250°C und besonders bevorzugt bei Temperaturen von 120 bis 240°C durchgeführt. Dabei soll die Flüssigphasentemperatur in den Rührbehältern nicht über der Verdampfungstemperatur der eingesetzten phenolischen Verbindung der Formel (III) liegen. Es kann deshalb von Vorteil sein, die erfindungsgemäße Umesterung im Bereich der Rührbehälter nicht nur bei Normaldruck, sondern auch bei erhöhtem oder erniedrigtem Druck im Bereich von 10 mbar bis 20 bar durchzuführen. Ein bevorzugter Druckbereich liegt zwischen 0,05 und 15 bar, ein besonders bevorzugter Druckbereich liegt zwischen 0,08 und 10 bar.

Katalysatoren, die für das erfindungsgemäße Verfahren in Frage kommen und die für alle Phasen des erfindungsgemäßen Verfahrens gleich sein können, sind in der Literatur bekannt. Solche Katalysatoren sind beispielsweise Hydride, Oxide, Hydroxide, Alkoholate, Amide oder Salze von (Erd)Alkalimetallen, wie Lithium, Natrium, Kalium, Rubidium, Cäsium, Magnesium und Calcium, bevorzugt von Lithium, Natrium, Kalium, Magnesium und Calcium, besonders bevorzugt von Lithium, Natrium und Kalium (US-3 642 858, US-3 803 201, EP 1082). Für den Fall des Einsatzes der Alkoholate können diese erfindungsgemäß auch in situ durch Einsatz der elementaren Alkalimetalle und des erfindungsgemäßen umzusetzenden Alkohols gebildet werden. Salze der (Erd)Alkalimetalle können solche von organischen oder anorganischen Säuren sein, wie von Essigsäure, Propionsäure, Buttersäure, Benzoesäure, Stearinsäure, Kohlensäure (Carbonate oder Hydrogencarbonate), von Salzsäure, Bromwasserstoff- oder Iodwasserstoffsäure, Salpetersäure, Schwefelsäure, Fluorwasserstoffsäure, Phosphorsäure, Blausäure, Rhodanwasserstoff, Borsäure, Zinnsäure, $C_1$-$C_4$-Stannonsäuren oder Antimonsäuren. In bevorzugter Weise kommen als Verbindungen der (Erd)Alkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt.

Solche (Erd)Alkalimetallverbindungen (gegebenenfalls in situ gebildet aus den freien Alkalimetallen) werden in Mengen von 0,001 bis 2 Gew.-%, bevorzugt 0,005 bis 0,9 Gew.-%, besonders bevorzugt 0,01 bis 0,5 Gew.-%, bezogen auf das umzusetzende Reaktionsgemisch, eingesetzt.

Weitere erfindungsgemäß einsetzbare Katalysatoren sind Lewis-saure Metallverbindungen wie $AlX_3$, $TiX_3$, $UX_4$, $TiX_4$, $VOX_3$, $VX_5$, $ZnX_2$, $FeX_3$ und $SnX_4$, worin X für Halogen, Acetoxy oder Aryloxy steht (DE-OS 2 528 412, 2 552 907), beispielsweise Titantetrachlorid, Titantetraphenoxid, Titantetraethoxid, Titantetraisopropylat, Titantetradodecylat, Zinntetraisooctylat und Aluminiumtriisopropylat, weiterhin zinnorganische Verbindungen der allgemeinen Formel $(R^4)_{4-x}$-$Sn(Y)_x$, in der Y für einen Rest $OCOR^5$, OH oder $OR^5$ steht, wobei $R^5$ $C_1$-$C_{12}$-Alkyl, $C_6$-$C_{12}$-Aryl oder $C_7$-$C_{13}$-Alkylaryl bedeutet und $R^4$ unabhängig von $R^5$ den Bedeutungsumfang von $R^5$ annehmen kann und x eine ganze Zahl von 1 bis 3 bedeutet, Dialkylzinnverbindungen mit 1 bis 12 C-Atomen im Alkylrest oder Bis(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew,-% (EP 879, EP 880, EP 39 452, DE-OS 3 445 555, JP 79/62 023), polymere Zinnverbindungen der Formel -[-$R^4$,$R^5$Sn-O-]-, beispielsweise Poly[oxy(dibutylstannylen)], Poly[oxy(dioctylstannylen)], Poly[oxy(butylphenylstannylen)] und Poly[oxy(diphenylstannylen)] (DE-OS 3 445 552), polymere Hydroxystannoxane der Formel -[$R^4$Sn(OH)-O-]-, beispielsweise Poly(ethylhydroxystannoxan), Poly(butylhydroxystannoxan), Poly(octylhydroxystannoxan), Poly(undecylhydroxystannoxan) und Poly(dodecylhydroxystannoxan) in Mengen von 0,001 bis 20 Gew.-%, bevorzugt von 0,005 bis 5 Gew.-%, bezogen auf Kohlensäurediester (DE 4 006 520). Weitere erfindungsgemäß einsetzbare Zinnverbindungen sind Sn(II)oxid oder besitzen die Formel

$$X^1\text{-}Sn(R^4)_2\text{-}O\text{-}Sn(R^4)_2\text{-}X^2 \hspace{4cm} (IX)$$

worin

$X^1$ und $X^2$      unabhängig voneinander OH, SCN, $OR^4$, $OCOR^4$ oder Halogen und
$R^4$             Alkyl, Aryl bedeutet (EP 338 760).

Als weitere erfindungsgemäß einsetzbare Katalysatoren kommen Bleiverbindungen, gegebenenfalls zusammen mit Triorganophosphanen, einer Chelatverbindung oder einem Alkalimetallhalogenid, beispielsweise $Pb(OH)_2 \cdot 2PbCO$, $Pb(OCO\text{-}CH_3)_2$, $Pb(OCO\text{-}CH_3)_2 \cdot 2LiCl$, $Pb(OCO\text{-}CH_3)_2 \cdot 2PPh_3$ in Mengen von 0,001 bis 1, bevorzugt von 0,005 bis 0,25 mol pro Mol Carbonat (JP 57/176 932, JP 01/093 580), andere Blei(II)- und Blei(IV)-verbindungen, wie PbO, $PbO_2$, Mennige, Plumbite ($PbO_2^{2-}$) und Plumbate ($PbO_3^{2-}$) (JP 01/093 560), Eisen(III)acetat (JP 61/172 852), weiterhin Kupfersalze und/oder Metallkomplexe, beispielsweise von Alkalimetallen, Zink, Titan und Eisen (JP 89/005 588), Kombinationen aus Lewis-Säuren und Protonensäuren (DE-OS 3 445 553) oder Elementverbindungen von Sc, Cr, Mo, W, Mn, Au, Ga, In, Bi, Te und Lanthaniden (EP 338 760) in Frage.

Weiterhin sind im erfindungsgemäßen Verfahren heterogene Katalysatorsysteme einsetzbar. Solche sind beispielsweise Mischoxide aus Silicium und Titan, die durch gemeinsame Hydrolyse von Silicium- und Titanhalogeniden herstellbar sind (JP 54/125 617) und Titandioxide mit hoher BET-Oberfläche > 20 m$^2$/g (DE-OS 4 036 594).

Bevorzugt im erfindungsgemäßen Verfahren einsetzbare Katalysatoren sind Zinn-, Titan- und Zirkoniumverbindungen und die obengenannten Alkali- und Erdalkaliverbindungen, besonders bevorzugt einsetzbare Katalysatoren sind Organozinnverbindungen und Titantetraalkoholate und -phenolate.

Die einzusetzenden Katalysatormengen betragen 0,01 bis 10 mol-%, bevorzugt 0,05 bis 5 mol-% und besonders bevorzugt 0,01 bis 2 mol-%, bezogen auf eingesetzte Phenol- oder Alkylarylcarbonatkomponente, und können sich teilweise von den in der Literatur genannten Mengen unterscheiden.

Die folgenden Beispiele sollen die vorliegende Erfindung konkret erläutern, wobei sie nicht auf diese Beispiele beschränkt sein soll.

Beispiele

Beispiel 1 (gemäß Fig. 2 ohne Rührbehälter E)

Drei mit Öl beheizte Doppelmantelgefäße mit jeweils 1 l Innenvolumen wurden so hintereinandergeschaltet, daß die Flüssigphase am Boden des ersten Reaktors zudosiert, aus dem ersten Reaktor über einen, in der Höhe verstellbaren, Siphon entnommen und am Boden des nächsten Reaktors wieder eingespeist wurde. Am dritten Reaktor wurde die Flüssigphase über einen Siphon entnommen. Die Gasphase wurde in den dritten Reaktor eingespeist, am Kopf wieder entnommen und in den vorgeschalteten Reaktor weitergeleitet. Die Vermischung von Gas- und Flüssigphase erfolgte in den einzelnen Reaktoren durch schnell laufende Begasungsrührer (1500 U/min). Die Entnahme der Gasphase erfolgte am Kopf des ersten Reaktors über eine 30 cm lange mit Raschigringen gefüllte Kolonne mit aufgesetztem Kolonnenkopf, der die Einstellung eines Rücklaufs auf die Kolonne erlaubte. Die Dosier- und Verbindungsleitungen waren mit Heizbändern so thermostatisiert, daß sowohl die Kristallisation der Flüssigphase als auch die Kondensation der Gasphase in diesen Leitungen verhindert wurde.

Die Reaktoren wurden mit jeweils 600 ml Phenol gefüllt und die Reaktormäntel mit Öl auf 180°C thermostatisiert. Über eine beheizte Pumpe wurden 510 g/h einer Mischung aus 98,6 Gew.-% Phenol und 1,4 Gew.-% Octylstannonsäure (Flüssigphase) in den ersten Reaktor kontinuierlich dosiert und gleichzeitig 500 g/h Dimethylcarbonat, das kontinuierlich in einem elektrisch beheizten Rohr verdampft wurde, in den dritten Reaktor. Nach 5 h befand sich die Reaktion im stationären Gleichgewicht, d.h. die Zusammensetzung der Flüssigphasen in den einzelnen Reaktoren und die Produktzusammensetzung änderte sich nicht mehr. Am dritten Reaktor wurden dann kontinuierlich 579 g/h Produktgemisch mit 60 g/h Methylphenylcarbonat und 12 g/h Diphenylcarbonat entnommen. Der Rest zu 100 % waren Phenol, wenig Dimethylcarbonat und Katalysator.

Am Kopf der am ersten Reaktor aufgesetzten Kolonne wurden 453 g/h Produktgemisch aus Methanol und Dimethylcarbonat entnommen. Daraus ergibt sich eine Raum-Zeit-Ausbeute für die Methylphenyl- und Diphenylcarbonatbildung von 24 g x $1^{-1}h^{-1}$. Die Selektivität bezüglich der Bildung aromatischer Carbonate war > 99,9 %.

Beispiel 2

In der in Beispiel 1 beschriebenen Apparatur (Fig. 2 ohne E) und unter den dort angegebenen Reaktionsbedingungen wurden 1,45 kg/h eines Gemisches aus 97,8 Gew.-% Phenol und 2,2 Gew.-% Titantetraphenolat am Boden des 1. Reaktors und 1,35 kg/h Dimethylcarbonat am Kopf des dritten Reaktors kontinuierlich eingespeist. Nach ca. 5 h befand sich die Reaktion im Gleichgewicht. Am Ende des 3. Reaktors wurden kontinuierlich 1,51 kg/h flüssiges Produktgemisch mit 175 g/h Methylphenylcarbonat und 42 g/h Diphenylcarbonat und am Kopf der am ersten Reaktor aufgesetzten Kolonne 1,27 kg/h Gemisch aus Methanol und Dimethylcarbonat entnommen. Das entspricht einer Raum-Zeit-Ausbeute für die Methylphenyl- und Diphenylcarbonatbildung von 72 g x $1^{-1}h^{-1}$. Die Selektivität bezgl. Bildung von aromatischen Carbonaten war > 99 %.

Beispiel 3

Das Beispiel 2 wurde in der dort angegebenen Apparatur und mit den dort angegebenen Reaktionsbedingungen und Eduktströmen wiederholt. Darüber hinaus wurde die am dritten Reaktor entnommene Flüssigphase kontinuierlich in einen weiteren Rührbehälter (E in Fig. 2) mit 2 l Innenvolumen und aufgesetzter Kolonne geleitet und dort kontinuierlich wieder über einen höhenverstellbaren und beheizten Siphon entnommen. Dieser Rührbehälter wurde auf 175°C Innentemperatur erhitzt. Nach 7 h befand sich die Reaktion im Gleichgewicht. Am 4. Rührbehälter wurden kontinuierlich 1,47 kg/h Flüssigphase mit 17,5 g/h Methylphenyl- und 157 g/h Diphenylcarbonat entnommen. Das entspricht einer Raum-Zeit-Ausbeute der Diphenylcarbonat- und Methylphenylcarbonatbildung von 35 g x $l^{-1}h^{-1}$. Die Selektivität der Bildung der aromatischen Carbonate war > 99 %.

Vergleichsbeispiel:

Ein beheizter Rührbehälter mit 2 l Innenvolumen, der mit einer 1,2 m langen, mit 6 mm Glasringen gefüllten Kolonne ausgestattet war, wurde mit 942 g Phenol und 21 g Titantetraphenolat gefüllt. Der Inhalt des Behälters wurde auf 175°C vorgeheizt und Dimethylcarbonat so zugetropft, daß die Innentemperatur zwischen 160 und 165°C gehalten wurde. Innerhalb von 4 h wurden 155 g Dimethylcarbonat zugetropft und gleichzeitig am Kopf der Kolonne ein Gemisch aus 17,4 g Methanol und 15,5 g Dimethylcarbonat entnommen. Das Sumpfprodukt hatte nach dieser Zeit die Zusammensetzung 854 g Phenol, 117 g Methylphenylcarbonat, 26 g Diphenylcarbonat, 89 g Dimethylcarbonat und 3 g Nebenprodukte. Daraus ergab sich eine Raum-Zeit-Ausbeute für Bildung der aromatischen Carbonate von ca. 18 g.l$^{-1}$h$^{-1}$. Die Selektivität der Arylcarbonatbildung betrug ca. 89 %.

**Patentansprüche**

1. Verfahren zur Herstellung eines aromatischen Carbonats der Formel

$$R^1\text{-O-CO-O-}R^2 \qquad (I)$$

in der

R$^2$    Phenyl oder Naphthyl sowie ein- bis dreifach durch geradkettiges oder verzweigtes $C_1$-$C_4$-Alkyl, geradkettiges oder verzweigtes $C_1$-$C_4$-Alkoxy, Cyano und/oder Halogen substituiertes Phenyl bzw. Naphthyl bedeutet, und

R$^1$    unabhängig von R$^2$ den Bedeutungsumfang von R$^2$ annimmt oder geradkettiges oder verzweigtes $C_1$-$C_6$-Alkyl bedeutet,

durch katalysierte Umsetzung von je 0,1-10 mol, bevorzugt 0,2-5 Mol, besonders bevorzugt 0,5-3 mol eines organischen Carbonats mit mindestens einer aliphatischen Estergruppe der Formel

$$R^1\text{-OCOO-}R^3 \qquad (II)$$

in der

R$^3$    geradkettiges oder verzweigtes $C_1$-$C_6$-Alkyl bedeutet und

R$^1$    den obigen Bedeutungsumfang hat,

mit 1 mol einer phenolischen Verbindung der Formel

$$R^2\text{-OX} \qquad (III)$$

in der

R$^2$    den obigen Bedeutungsumfang hat und
X     für Wasserstoff oder für -CO-O-$C_1$-$C_6$-Alkyl mit geradkettiger oder verzweigter Alkylgruppe steht,

in Gegenwart eines an sich bekannten Umesterungskatalysators bei 80-350°C und 10 mbar bis 20 bar, dadurch gekennzeichnet, daß die Umsetzung in mindestens zwei hintereinandergeschalteten Rührbehältern nach dem Prinzip des Querstroms oder des Gegenstroms so durchgeführt wird, daß die phenolische Verbindung der Formel (III) in flüssiger Form in den ersten Rührbehälter und das organische Carbonat der Formel (II) bei Querstromfahrweise in jeden der Rührbehälter und bei Gegenstromfahrweise in den letzten Rührbehälter eindosiert wird und am letzten Rührbehälter, gegebenenfalls nach Durchlaufen einer Verweilzeitstrecke, das Reaktionsprodukt der Formel (I) in flüssiger Form und die Produkte der Formel

$$R^3\text{-OX} \qquad (IV)$$

in der

R$^3$ und X    die genannte Bedeutung haben,

bei Querstromfahrweise am Kopf jedes Rührbehälters und bei Gegenstromfahrweise am Kopf des ersten Rührbehälters entnommen werden.

2. Verfahren nach Anspruch 1, dadurch gekennzeichnet, daµ die Umsetzung in mindestens zwei hintereinandergeschalteten Rührbehältern so durchgeführt wird, daß das organische Carbonat der Formel (II) in den letzten Rührbehälter eindosiert wird und am letzten Rührbehälter das aromatische Carbonat der Formel (I) in flüssiger Form und am Kopf des ersten Rührbehälters das Produkt der Formel (IV) entnommen wird.

**3.** Verfahren nach Anspruch 1, dadurch gekennzeichnet, daß die Umsetzung in 2 bis 10, bevorzugt in 3 bis 10, besonders bevorzugt in 3 bis 8, hintereinandergeschalteten Rührbehältern durchgeführt wird.

**4.** Verfahren nach Anspruch 1, dadurch gekennzeichnet, daß bei 100-250°C, bevorzugt bei 120 bis 240°C gearbeitet wird.

**5.** Verfahren nach Anspruch 1, dadurch gekennzeichnet, daß im Druckbereich von 0,05 bis 15 bar, bevorzugt 0,08 bis 10 bar gearbeitet wird.

**6.** Verfahren nach Anspruch 1, dadurch gekennzeichnet, daß eine phenolische Verbindung der Formel

$$R^{12}\text{-OH} \qquad\qquad (V)$$

eingesetzt wird,
in der

$R^{12}$    Phenyl oder einfach durch $C_1$-$C_4$-Alkyl, $C_1$-$C_4$-Alkoxy oder Chlor substituiertes Phenyl bedeutet.

**7.** Verfahren nach Anspruch 6, dadurch gekennzeichnet, daß nicht substituiertes Phenol eingesetzt wird.

**8.** Verfahren nach Anspruch 1, dadurch gekennzeichnet, daß ein symmetrisches Dialkylcarbonat der Formel

$$R^3\text{-O-CO-O-}R^3 \qquad\qquad (VI)$$

in der

$R^3$    geradkettiges oder verzweigtes $C_1$-$C_6$-Alkyl bedeutet,

als mindestens eine aliphatische Estergruppe enthaltendes organisches Carbonat eingesetzt wird.

**9.** Verfahren nach Anspruch 8, dadurch gekennzeichnet, daß Dimethylcarbonat eingesetzt wird.

**10.** Verfahren nach Anspruch 1, dadurch gekennzeichnet, daß das organische Carbonat (II) im Gemisch mit 0-5 Gew.-%, bevorzugt 0,1-3 Gew.-%, besonders bevorzugt 0,15-2 Gew.-%, bezogen auf das Gewicht von (II), an zugrundeliegendem Alkohol $R^3$-OH eingesetzt wird.

**Claims**

**1.** Process for producing an aromatic carbonate of the formula

$$R^1\text{-O-CO-O-}R^2 \qquad\qquad (I)$$

in which

$R^2$    is phenyl or naphthyl and phenyl or naphthyl mono- to trisubstituted by straight-chain or branched $C_1$-$C_4$-alkyl, straight-chain or branched $C_1$-$C_4$-alkoxy, cyano and/or halogen, and

$R^1$    assumes, independently of $R^2$, the scope of meaning of $R^2$ or straight-chain or branched $C_1$-$C_6$-alkyl,

by catalysed reaction of, in each case, 0.1-10 mol, preferably 0.2-5 mol, particularly preferably 0.5-3 mol of an organic carbonate containing at least one aliphatic ester group of the formula

$$R^1\text{-OCOO-}R^3 \qquad\qquad (II)$$

in which

$R^3$    is straight-chain or branched $C_1$-$C_6$-alkyl and
$R^1$    has the above scope of meaning,

with 1 mol of a phenolic compound of the formula

$$R^2\text{-OX} \hspace{4cm} \text{(III)}$$

in which

$R^2$     has the above scope of meaning and

X     represents hydrogen or $-CO-O-C_1-C_6$-alkyl containing a straight-chain or branched alkyl group,

in the presence of a transesterification catalyst, known per se at, 80-350°C and 10 mbar to 20 bar, characterized in that the reaction is carried out in at least two stirred containers connected one behind the other in accordance with the principle of cross flow or counter flow in such a way that the phenolic compound of the formula (III) is metered in liquid form into the first stirred container and the organic carbonate of the formula (II) in cross-flow mode into each of the stirred containers and in counter-flow mode into the last stirred container and the reaction product of the formula (I) is removed in liquid form at the last stirred container, if necessary after traversing a dwell-time section, and the products of the formula

$$R^3\text{-OX} \hspace{4cm} \text{(IV)}$$

in which

$R^3$ and X     have the specified meaning

are removed in cross-flow mode at the head of each stirred container and in counter-flow mode at the head of the first stirred container.

2.   Process according to Claim 1, characterized in that the reaction is carried out in at least two stirred containers connected one behind the other in such a way that the organic carbonate of the formula (II) is metered into the last stirred container and the aromatic carbonate of the formula (I) is removed in liquid form at the last stirred container and the product of the formula (IV) is removed at the head of the first stirred container.

3.   Process according to Claim 1, characterized in that the reaction is carried out in 2 to 10, preferably in 3 to 10, particularly preferably in 3 to 8, stirred containers connected one behind the other.

4.   Process according to Claim 1, characterized in that the process is carried out at 100-250°C, preferably at 120 to 240°C.

5.   Process according to Claim 1, characterized in that the process is carried out in the pressure range from 0.05 to 15 bar, preferably 0.08 to 10 bar.

6.   Process according to Claim 1, characterized in that a phenolic compound of the formula

$$R^{12}\text{-OH} \hspace{4cm} \text{(V)}$$

is used,
in which

$R^{12}$     is phenyl or phenyl monosubstituted by $C_1-C_4$-alkyl, $C_1-C_4$-alkoxy or chlorine.

7.   Process according to Claim 6, characterized in that unsubstituted phenol is used.

8.   Process according to Claim 1, characterized in that a symmetrical dialkyl carbonate of the formula

$$R^3\text{-O-CO-O-}R^3 \hspace{4cm} \text{(VI)}$$

in which

$R^3$     is straight-chain or branched $C_1-C_6$-alkyl, is used as organic carbonate containing at least one aliphatic ester group.

9. Process according to Claim 8, characterized in that dimethyl carbonate is used.

10. Process according to Claim 1, characterized in that the organic carbonate (II) is used in a mixture with 0-5 % by weight, preferably 0.1-3 % by weight, particularly preferably 0.15-2 % by weight, based on the weight of (II), of basic alcohol $R^3$-OH.

**Revendications**

1. Procédé pour la préparation d'un carbonate aromatique répondant à la formule

$$R^1\text{-O-CO-O-}R^2 \qquad (I)$$

dans laquelle

$R^2$ représente un groupe phényle ou un groupe naphtyle, ainsi qu'un groupe phényle, respectivement un groupe naphtyle portant de un à trois substituants alkyle en $C_1$-$C_4$ à chaîne droite ou ramifiée, alcoxy en $C_1$-$C_4$ à chaîne droite ou ramifiée, cyano et/ou halogéno, et

$R^1$ indépendamment de $R^2$, prend la portée de signification de $R^2$ ou représente un groupe alkyle en $C_1$-$C_6$ à chaîne droite ou ramifiée,

par mise en réaction catalytique de respectivement 0,1-10 moles, de préférence de 0,2-5 moles, de manière particulièrement préférée de 0,5-3 moles d'un carbonate organique comprenant au moins un groupe ester aliphatique de formule

$$R^1\text{-OCOO-}R^3 \qquad (II)$$

dans laquelle

$R^3$ représente un groupe alkyle en $C_1$-$C_6$ à chaîne droite ou ramifiée et

$R^1$ a la portée de signification indiquée ci-dessus,

avec 1 mole d'un composé phénolique répondant à la formule

$$R^2\text{-OX} \qquad (III)$$

dans laquelle

$R^2$ a la portée de signification indiquée ci-dessus, et

X représente un atome d'hydrogène ou encore un groupe -CO-O-alkyle en $C_1$-$C_6$ contenant un groupe alkyle à chaîne droite ou ramifiée,

en présence d'un catalyseur de trans-estérification connu en soi, à une température de 80 à 350°C et sous une pression de 10 mbar à 20 bar, caractérisé en ce qu'on effectue la mise en réaction dans au moins deux récipients d'agitation montés l'un derrière l'autre conformément au principe du courant transversal ou du contre-courant, en ce que le composé phénolique de formule (III) est introduit de manière dosée sous forme liquide dans le premier récipient d'agitation et le carbonate organique de formule (II) est introduit de manière dosée dans un mode opératoire à courant transversal dans chacun des récipients d'agitation et dans un mode opératoire à contre-courant dans le dernier récipient d'agitation, et on prélève du dernier récipient d'agitation, éventuellement après son passage par un tronçon de temps de séjour, le produit réactionnel de formule (I) sous forme liquide et les produits de formule

$$R^3\text{-OX} \qquad (IV)$$

dans laquelle

$R^3$ et X ont la signification mentionnée,

dans un mode opératoire à courant transversal à la tête de chaque récipient d'agitation et dans un mode opératoire à contre-courant à la tête du premier récipient d'agitation.

2. Procédé selon la revendication 1, caractérisé en ce qu'on effectue la mise en réaction dans au moins deux récipients d'agitation montés l'un derrière l'autre de telle sorte que l'on introduit par dosage le carbonate organique de formule (II) dans le dernier récipient d'agitation et on prélève du dernier récipient d'agitation le carbonate aromatique de formule (I) sous forme liquide et le produit de formule (IV) à la tête du premier récipient d'agitation.

3. Procédé selon la revendication 1, caractérisé en ce qu'on effectue la mise en réaction dans 2 à 10, de préférence dans 3 à 10, de manière particulièrement préférée dans 3 à 8 récipients d'agitation montés l'un derrière l'autre.

4. Procédé selon la revendication 1, caractérisé en ce qu'on travaille à une température de 100 à 250°C, de préférence de 120 à 240°C.

5. Procédé selon la revendication 1, caractérisé en ce qu'on travaille dans le domaine de pression de 0,05 à 15 bar, de préférence de 0,08 à 10 bar.

6. Procédé selon la revendication 1, caractérisé en ce qu'on met en oeuvre un composé phénolique de formule

$$R^{12}\text{-OH} \qquad\qquad (V)$$

dans laquelle

$R^{12}$ représente un groupe phényle ou encore un groupe phényle portant un substituant alkyle en $C_1$-$C_4$, alcoxy en $C_1$-$C_4$ ou chloro.

7. Procédé selon la revendication 6, caractérisé en ce qu'on met en oeuvre du phénol non substitué.

8. Procédé selon la revendication 1, caractérisé en ce qu'on met en oeuvre un dialkylcarbonate symétrique répondant à la formule

$$R^3\text{-O-CO-O-}R^3 \qquad\qquad (VI)$$

dans laquelle

$R^3$ représente un groupe alkyle en $C_1$-$C_6$ à chaîne droite ou ramifiée,

à titre de carbonate organique contenant au moins un groupe ester aliphatique.

9. Procédé selon la revendication 8, caractérisé en ce qu'on met en oeuvre du diméthylcarbonate.

10. Procédé selon la revendication 1, caractérisé en ce qu'on met en oeuvre le carbonate organique (II) en mélange avec de 0 à 5% en poids, de préférence de 0,1 à 3% en poids, de manière particulièrement préférée de 0,15 à 2% en poids rapportés au poids de (II), de l'alcool de départ $R^3$-OH.

F i g.1

Fig.2